# EUROPEAN PATENT APPLICATION

(11) **EP 4 806 442 A1**
(43) Date of publication of application: **16.09.2026**
(21) Application number: 24906913.9
(22) Date of filing: 23.10.2024
(51) Int. Cl.: A61K 33/00, A61P 1/00, C02F 1/461

(54) **ELECTROLYZED HYDROGEN WATER, METHOD FOR PRODUCING SAME, METHOD FOR ACTIVATING INTESTINAL BARRIER MAINTENANCE FUNCTION USING SAME, AND METHOD FOR REGULATING GENE EXPRESSION USING SAME**

(30) Priority: 22.12.2023 JP 2023216875
(71) Applicant: Nihon Trim Co., Ltd., Osaka 531-0076 (JP)
(72) Inventor: HARA Taichi, Tokyo 169-8050 (JP); KABAYAMA Shigeru, Osaka-shi, Osaka 531-0076 (JP)
(74) Representative: Lorenz Seidler Gossel Part. mbB
(86) International application number: PCT/JP2024/037805
(87) International publication number: WO 2025/134525

(57) **Abstract**

Electrolyzed hydrogen water has activity for enhancing an intestinal barrier maintenance function by regulating the expression of a responsive gene, and the responsive gene is at least one hub gene selected from the group consisting of CUL5 (Cullin 5), GOLGA7 (Golgin A7), has-miR-429, and has-miR-200c-3p. A method for regulating gene expression using electrolyzed hydrogen water (excluding medical practice to humans) includes: adjusting a dissolved hydrogen concentration to 1,056 ppb or more and 1,080 ppb or less; and increasing the expression of CUL5 (Cullin 5) and GOLGA7 (Golgin A7) and decreasing the expression of has-miR-429 and has-miR-200c-3p by the action of the electrolyzed hydrogen water.

## Description

### TECHNICAL FIELD

The present invention relates to electrolyzed hydrogen water, a method for producing the same, a method for activating intestinal barrier maintenance function using the same, and a method for regulating gene expression using the same.

### BACKGROUND ART

The present applicant has made various studies on new applications and functions of various types of hydrogen water such as electrolyzed hydrogen water and mixtures containing the same. For example, Patent Document 1 proposes a hydrogen water mixture for suppressing alcoholic liver disease, in which an ethanol solvent and hydrogen water are mixed with an ethanol concentration of 1% to 4% and a dissolved hydrogen concentration of 550 ppb to 5,600 ppb. The hydrogen water mixture is highly safe, easy and inexpensive to prepare, and can effectively suppress the alcoholic liver disease.

### CITATION LIST

### PATENT DOCUMENT

Patent Document 1: Japanese Unexamined Patent Publication No. 2022-126128

### SUMMARY OF THE INVENTION

### TECHNICAL PROBLEM

Some genes are activated when electrolyzed hydrogen water acts on cells (the genes may be hereinafter referred to as "responsive genes" that respond to the electrolyzed hydrogen water). If the responsive genes are identified, it is expected that the expression of the responsive genes is regulated by the electrolyzed hydrogen water. Regulating the expression of the responsive genes by the electrolyzed hydrogen water enables the electrolyzed hydrogen water to exhibit the biological functions of the responsive genes.

The present invention has been made in view of the above circumstances, and an object of the present invention is to provide electrolyzed hydrogen water having activity for enhancing biological functions of a responsive gene by regulating the expression of the responsive gene, and a method for using the electrolyzed hydrogen water.

### SOLUTION TO THE PROBLEM

As a result of intensive studies to achieve the object, the present inventors have identified four responsive genes that act on the enhancement of an intestinal barrier maintenance function through immune regulation as hub genes responding to electrolyzed hydrogen water, and have completed the electrolyzed hydrogen water of the present invention.

The electrolyzed hydrogen water of the present invention has activity for enhancing an intestinal barrier maintenance function by regulating the expression of a responsive gene. The responsive gene is at least one hub gene selected from the group consisting of CUL5 (Cullin 5), GOLGA7 (Golgin A7), has-miR-429, and has-miR-200c-3p.

A method for activating an intestinal barrier maintenance function according to the present invention is a method for enhancing and activating an intestinal barrier maintenance function by regulating the expression of a responsive gene using electrolyzed hydrogen water. The responsive gene is at least one hub gene selected from the group consisting of CUL5 (Cullin 5), GOLGA7 (Golgin A7), has-miR-429, and has-miR-200c-3p, and the method includes adjusting a concentration of dissolved hydrogen to 1,056 ppb or more and 1,080 ppb or less.

A method for producing electrolyzed hydrogen water for activating an intestinal barrier maintenance function according to the present invention is a method for producing electrolyzed hydrogen water that enhances and activates an intestinal barrier maintenance function by regulating the expression of a responsive gene. The responsive gene is at least one hub gene selected from the group consisting of CUL5 (Cullin 5), GOLGA7 (Golgin A7), has-miR-429, and has-miR-200c-3p, and the method includes adjusting a concentration of dissolved hydrogen to 1,056 ppb or more and 1,080 ppb or less.

A method for regulating gene expression using electrolyzed hydrogen water according to the present invention is a method for regulating gene expression using electrolyzed hydrogen water (excluding medical practice to humans). A gene responsive to the electrolyzed hydrogen water is at least one hub gene selected from the group consisting of CUL5 (Cullin 5), GOLGA7 (Golgin A7), has-miR-429, and has-miR-200c-3p, and the method includes: adjusting a concentration of dissolved hydrogen to 1,056 ppb or more and 1,080 ppb or less; and increasing the expression of CUL5 (Cullin 5) and GOLGA7 (Golgin A7) and decreasing the expression of has-miR-429 and has-miR-200c-3p by the action of the electrolyzed hydrogen water.

### ADVANTAGES OF THE INVENTION

The present invention provides electrolyzed hydrogen water having activity for enhancing biological functions of a responsive gene by regulating the expression of the responsive gene, and a method for using the electrolyzed hydrogen water.

### BRIEF DESCRIPTION OF THE DRAWINGS

[FIG. 1] FIG. 1 is a diagram showing changes in gene expression by electrolyzed hydrogen water in human colorectal cancer cells (Caco-2 cells), with filtered water as a reference.
[FIG. 2] FIG. 2 is a diagram showing the amount of gene expression changes by the electrolyzed hydrogen water in the Caco-2 cells, with filtered water as a reference.
[FIG. 3] FIG. 3 is a diagram showing the results of a physiological function analysis based on Gene Ontology from the gene expression changes by the electrolyzed hydrogen water in the Caco-2 cells.
[FIG. 4] FIG. 4 is a diagram showing the results of an analysis of transcription factors regulating the expression of genes based on the changes in the expression of the genes by the electrolyzed hydrogen water in the Caco-2 cells.
[FIG. 5] FIG. 5 is a diagram showing changes in microRNA expression by the electrolyzed hydrogen water in the Caco-2 cells, with filtered water as a reference.
[FIG. 6] FIG. 6 is a diagram showing the amount of microRNA expression changes by the electrolyzed hydrogen water in the Caco-2 cells, with filtered water as a reference.
[FIG. 7] FIG. 7 is a diagram showing the results of an analysis of a network between differentially expressed genes and microRNAs in terms of protein interaction, for genes targeted by microRNAs whose expression is changed by the electrolyzed hydrogen water in the Caco-2 cells.
[FIG. 8] FIG. 8 is a diagram showing the results of an analysis of transcription factors common to hub genes responding to the electrolyzed hydrogen water in the Caco-2 cells.

### DESCRIPTION OF EMBODIMENTS

The present inventors screened for the differential expression of genes responding to electrolyzed hydrogen water based on the results of an RNA sequence (RNA-seq) analysis described in Examples below, and found the biological functions of the identified genes by a gene ontology (GO) analysis and an intermolecular interaction network analysis. The electrolyzed hydrogen water of the present invention has been completed based on these findings.

Hub genes that respond to the electrolyzed hydrogen water are CUL5 (Cullin 5), GOLGA7 (Golgin A7), has-miR-429, and has-miR-200c-3p (may be hereinafter collectively referred to as "specific responsive genes"). A responsive gene that responds to the electrolyzed hydrogen water of the present invention is specified as at least one hub gene selected from the group consisting of CUL5 (Cullin 5), GOLGA7 (Golgin A7), has-miR-429, and has-miR-200c-3p (the electrolyzed hydrogen water may be hereinafter referred to as "responsive gene-specific electrolyzed hydrogen water").

The expression of CUL5 (Cullin 5) and GOLGA7 (Golgin A7) is increased by the action of the electrolyzed hydrogen water. On the other hand, the expression of has-miR-429 and has-miR-200c-3p is decreased by the action of the electrolyzed hydrogen water. That is, the expression levels of the specific responsive genes can be regulated by using the electrolyzed hydrogen water.

A transcription factor KLF5 targets CUL5 (Cullin 5), GOLGA7 (Golgin A7), has-miR-429, and has-miR-200c-3p. It is considered that the specific responsive genes to the responsive gene-specific electrolyzed hydrogen water in the intestinal epithelium-like cells are controlled by KLF5.

The regulation of the expression of the specific responsive genes by the electrolyzed hydrogen water acts on the enhancement of one of the biological functions of the specific responsive genes, that is, an intestinal barrier maintenance function through immune regulation. In other words, the responsive gene-specific electrolyzed hydrogen water has activity for enhancing the intestinal barrier maintenance function by increasing or decreasing the expression of the specific responsive genes.

For example, the following papers describe that the specific responsive genes influence the intestinal function.
1) Yu, T., et al., Overexpression of miR-429 impairs intestinal barrier function in diabetic mice by down-regulating occludin expression. Cell Tissue Res, 2016. 366(2): pp. 341-352.
2) He, S., et al., Ferulic Acid Ameliorates Lipopolysaccharide-Induced Barrier Dysfunction via MicroRNA-200c-3p-Mediated Activation of PI3K/AKT Pathway in Caco-2 Cells. Front Pharmacol, 2020. 11: p. 376.
3) Mo, J. S., et al., MicroRNA 429 Regulates Mucin Gene Expression and Secretion in Murine Model of Colitis. J Crohns Colitis, 2016. 10(7): pp. 837-49.
4) Chen, J., et al., The roles of miR-200c in colon cancer and associated molecular mechanisms. Tumour Biol, 2014. 35(7): pp. 6475-83.

In addition, the responsive gene-specific electrolyzed hydrogen water is expected to control activation or inhibition of autophagy and prevent cancer progression by enhancing the intestinal barrier maintenance function.

Autophagy is an intracellular recycling system (a lysosome-dependent degradation system) that degrades intracellular components to purify the intracellular components and remove harmful substances, and acts to prevent various diseases such as carcinogenesis. It is also known that autophagy not only functions as a mechanism for degrading the intracellular components, but also plays a role in regulating biological mechanisms by the secretion of the intracellular components. In particular, it has been found that secretory autophagy (exophagy, extracellular release of secretions) is involved in inflammation regulation and metabolite exchange which are associated with various diseases.

The miR-200c family has been reported to have a potential impact on the progression of cancer. The overexpression of miR-200c promotes proliferation, migration, and invasion of cancer. On the other hand, it has been demonstrated that decreased expression of miR-200c inhibits proliferation, migration, and invasion of cancer. Thus, the responsive gene-specific electrolyzed hydrogen water, which suppresses the expression of the specific responsive gene hsa-miR-200c-3p, not only acts on the enhancement of the intestinal barrier maintenance function through immune regulation, but may possibly function to prevent cancer.

It is also reported that the decreased expression of hsa-miR-200c-3p promotes adhesion of cells to fibronectin, which is one of extracellular matrices forming a scaffold. Thus, the responsive gene-specific electrolyzed hydrogen water may be able to maintain the intercellular adhesion by suppressing the expression of the specific responsive gene hsa-miR-200c-3p.

The miR-429 family is known as a member of the miR-200 family and has similar functions. Thus, it is considered that the responsive gene-specific electrolyzed hydrogen water functions to maintain the intestinal barrier function by suppressing the expression of the specific responsive gene has-miR-429. It has been reported in in vivo studies in mice that miR-429 impairs the intestinal barrier function by decreasing the expression of genes involved in the barrier function.

It has been demonstrated that the increased expression of miR-200c or miR-429 promotes cellular inflammatory responses in diabetic mouse models.

The responsive gene-specific electrolyzed hydrogen water can be produced using a commercially available hydrogen generator (e.g., an electrolyzed water generator). Thus, it can be said that the responsive gene-specific electrolyzed hydrogen water is highly safe, and easy and inexpensive to prepare with a commercially available device.

The concentration of dissolved hydrogen in the responsive gene-specific electrolyzed hydrogen water is preferably 550 ppb or more and 5,600 ppb or less, more preferably 800 ppb or more and 1,320 ppb or less, and much more preferably 1,056 ppb or more and 1,080 ppb or less, from the viewpoint of regulating the expression of the specific responsive genes.

The responsive gene-specific electrolyzed hydrogen water configured as described above regulates the expression of at least one hub gene selected from the group consisting of CUL5 (Cullin 5), GOLGA7 (Golgin A7), has-miR-429, and has-miR-200c-3p, which are specified as the responsive genes, and thus has activity for enhancing the intestinal barrier maintenance function, which is the biological function of the hub genes.

### [Examples]

The present disclosure will be described below based on the Examples. The present invention is not limited to these Examples, and these Examples can be modified and changed in accordance with the spirit of the present invention. Such modifications and changes are not excluded from the scope of the present invention.

### <Production of Electrolyzed Hydrogen Water>

Electrolyzed hydrogen water with a dissolved hydrogen concentration level of 4 was obtained by using an electrolyzed water generator (manufactured by Nihon Trim Co., Ltd., trade name: TRIM ION GRACE) under the conditions of a temperature of 22°C and a flow rate of 1.5 L/min, and filtered water was obtained by filtration with micro carbon.

Next, the pH and dissolved hydrogen concentration of the electrolyzed hydrogen water of level 4 were measured. A pH meter (manufactured by HORIBA, Ltd., trade name: LAQUAact D-71) was used for the measurement of pH, and a dissolved hydrogen meter DH-35A (manufactured by DKK-TOA CORPORATION) was used for the measurement of the dissolved hydrogen concentration.
- Level 4: 1,320 ppb (to 1,350 ppb), pH 10.

### <Preparation of Medium for Treating Electrolyzed Hydrogen Water (Electrolyzed Hydrogen Water Medium)>

5 × Dulbecco's modified Eagle's media (hereinafter referred to as "DMEMs," Wako, 044-29765) each prepared from powder were diluted 5-fold with the filtered water or the electrolyzed hydrogen water of level 4, and used as media for treating the electrolyzed hydrogen water (i.e., hydrogen water mixtures as treatment media obtained by mixing 20% 5 × DMEM with 80% electrolyzed hydrogen water or filtered water). The dissolved hydrogen concentration in the electrolyzed hydrogen water medium of level 4 was as follows.
- Level 4: 1,320 ppb (to 1,350 ppb) × 4/5 = 1,056 ppb (to 1,080 ppb)

### <Cell Culturing>

Caco-2 cells (Riken BRC), which were a human colorectal adenocarcinoma cell line, were cultured in an electrolyzed hydrogen water medium containing 10% fetal bovine serum (FBS; Sigma-Aldrich, F7524) and 1% penicillin-streptomycin (Wako, 168-23191) at 37°C in a 5% CO₂ atmosphere.

### <Transcriptome Sequencing>

Samples extracted from six independent wells were used for an RNA-seq analysis. A cDNA library was constructed using NEB Next (registered trademark) Ultra^{™} RNA Library Prep Kit for Illumina (registered trademark) (New England Biolabs). The final cDNA library was prepared through several rounds of purification, end-repair, A-tailing, sequence adapter ligation, size selection, and PCR amplification. RNA sequencing was performed on the library on the NovaSeq 6000 System from Novogene Co. Ltd. Reads were mapped to the reference sequence using TopHat2. For transcriptome sequencing, three samples were randomly selected from an ACW group and an EHW-exposure group. A TRIzol^{™} reagent (Invitrogen) was used to extract total RNA from thymus specimens. Total RNA was sequenced using NEB Next Multiplex Directional RNA Library Prep Kit and NEB Small RNA Library Kit. The total RNA was quantified and identified, and then used as an input material for the synthesis of mRNA and microRNA libraries. All transcriptome sequence analysis was performed on the Illumina HiSeq^{™} 2500 platform.

### <Differentially Expressed Gene Analysis>

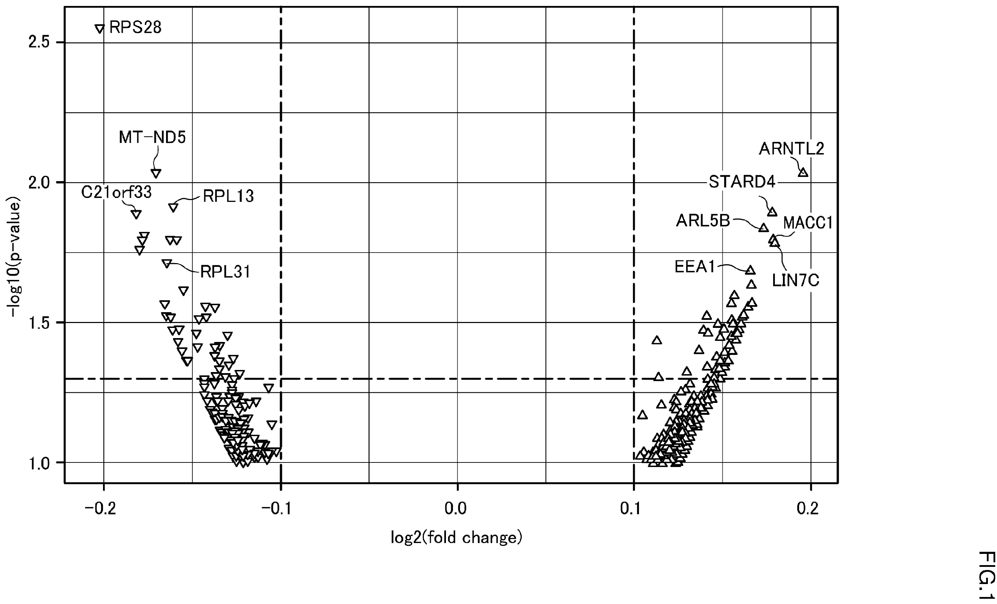

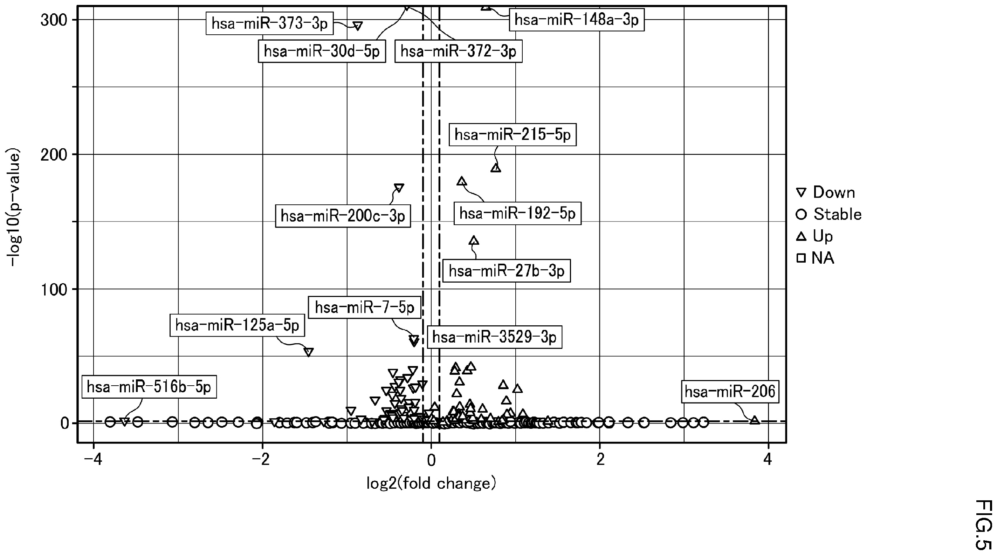

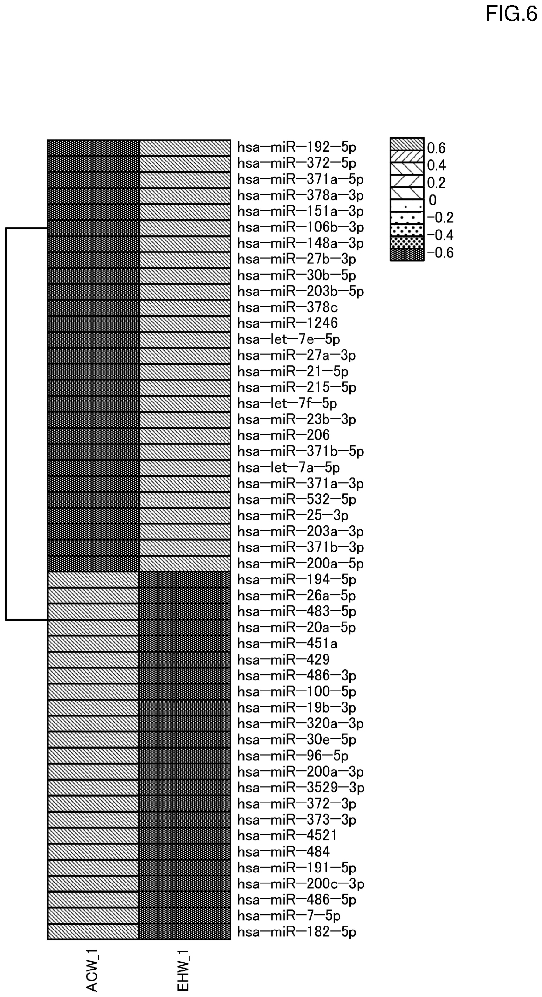

Differentially expressed genes were screened using an EBSeq toolkit and identified with FDR < 0.05 by the Benjamin-Hochberg method. FIGS. 1 to 4 show the results. FIGS. 5 and 6 show the results obtained using microRNAs.

FIG. 1 is a volcano plot showing changes in gene (mRNA) expression by electrolyzed hydrogen water (EHW) in human colorectal cancer cells (Caco-2 cells), with filtered water (ACW) as a reference. The vertical axis in FIG. 1 indicates a statistical significance level, and a higher value indicates higher statistical significance. The horizontal axis of FIG. 1 indicates the expression level of gene (mRNA) expression by the electrolyzed hydrogen water, and numerical values smaller than 0 represent genes with decreased expression levels, while numerical values larger than 0 represent genes with increased expression levels. From FIG. 1, 82 differentially expressed genes (DEGs) were extracted from the filtered water and the electrolyzed hydrogen water (extracted with p < 0.05 in False Discovery Rate: FDR). Of the 82 genes, 44 genes are differentially expressed genes (ARNTL2, MACC1, LIN7C, EEA1, ARL5B, STARD4, etc.) showing increased gene expression levels (increased mRNA levels). On the other hand, 38 genes are differentially expressed genes (RPS28, MT-ND5, RPL13, RPL31, C21orf33, etc.) showing decreased gene expression levels (decreased mRNA levels).

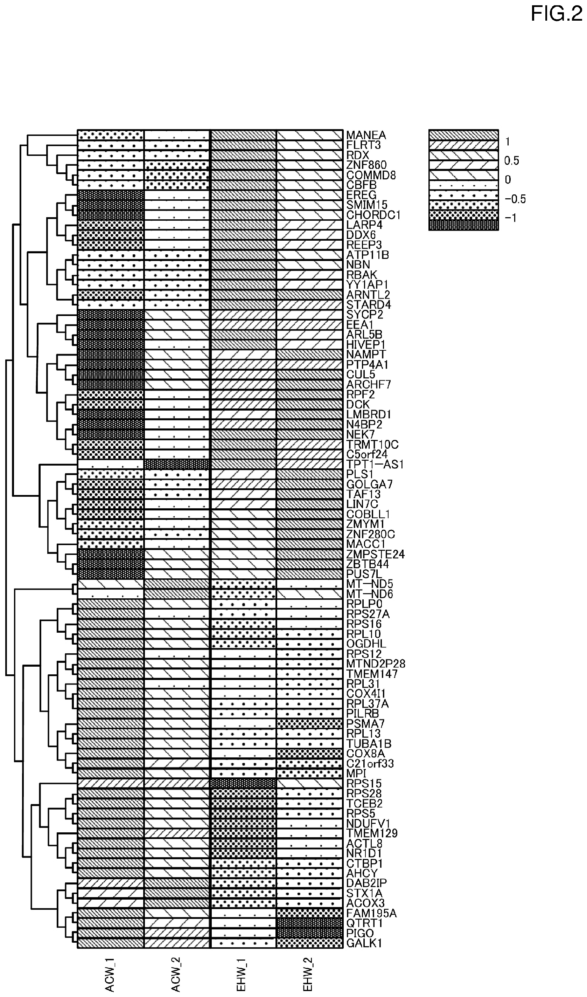

FIG. 2 is a heatmap showing, by color shading, the amount (degree) of gene expression changes caused by the electrolyzed hydrogen water (EHW) in human colorectal cancer cells (Caco-2 cells), with filtered water (ACW) as a reference. The heatmap compared two sets of independent experimental data by normalization. Alphabetical letters in FIG. 2 represent gene names. In FIG. 2, the genes whose expression was increased by the electrolyzed hydrogen water (MANEA to PUS7L) are shown in the upper part of the table, while the genes whose expression was decreased by the electrolyzed hydrogen water (MT-ND5 to GALK1) are shown in the lower part.

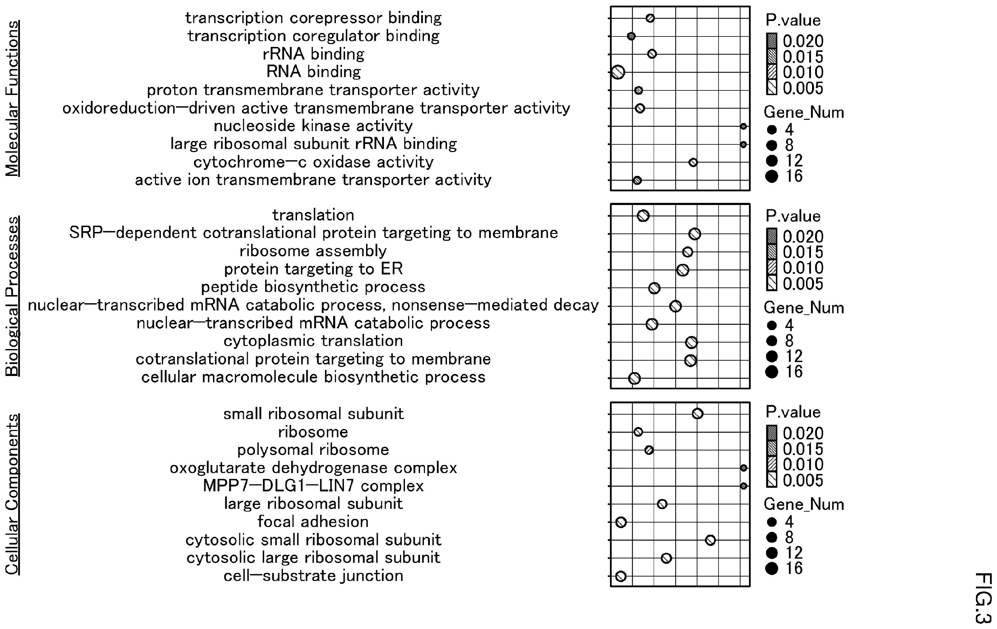

FIG. 3 shows the analysis of biological functions based on Gene Ontology (GO) in human colorectal cancer cells (Caco-2 cells). Specifically, FIG. 3 shows the results of an analysis of physiological functions (molecular functions, biological processes, and cellular components in order from the top of FIG. 3) based on GO from the gene expression changes by the electrolyzed hydrogen water (EHW). The GO analysis used the clusterProfiler package, which is an application for statistical analysis software in the R programming language. In the GO analysis, the top ten genes with the lowest p values < 0.05 were selected for visualization. As shown in FIG. 3, among the genes that had the expression changed by the electrolyzed hydrogen water, GO terms (functional terms) related to protein synthesis, transport, and intercellular adhesion were profiled at higher ranks. Specifically, examples of the GO terms of protein synthesis include rRNA binding, large ribosomal subunit rRNA binding, translation, ribosome assembly, peptide biosynthetic process, cytoplasmic translation, cellular macromolecule biosynthetic process, small ribosomal subunit, ribosome, polysomal ribosome, large ribosomal subunit, and cytosolic small ribosomal subunit. Examples of the GO terms of transport include proton transmembrane transporter activity, oxidoreduction-driven active transmembrane transporter activity, and active ion transmembrane transporter activity. Examples of the GO terms of intercellular adhesion include focal adhesion and cell-substrate junction.

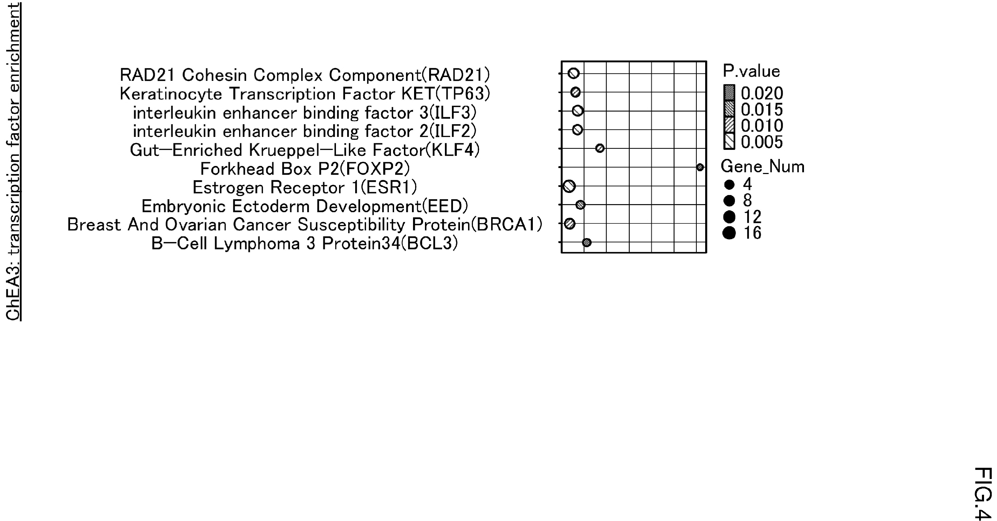

FIG. 4 shows the results of an analysis (enrichment analysis, ChIP-X Enrichment Analysis 3: ChEA3) of transcription factors that regulate differentially expressed genes (DEG) in human colorectal cancer cells (Caco-2 cells). Specifically, FIG. 4 shows the results of an analysis of transcription factors that regulate the expression of genes based on the changes in the expression of the genes by the electrolyzed hydrogen water (EHW). The ChIP-X Enrichment Analysis 3 (ChEA3) was used for the enrichment analysis of the transcription factors. In the ChEA3 analysis, the top ten genes with the lowest p values < 0.05 were selected for visualization. As shown in FIG. 4, a transcription factor associated with the enhancement of a skin barrier function (intestinal barrier function), namely, Gut-Enriched Krueppel-Like Factor (KLF4), was enriched as a transcription factor targeting the genes differentially expressed by the electrolyzed hydrogen water. KLF4 is also suggested to function as a colorectal cancer suppressor.

FIG. 5 is a volcano plot showing the changes in microRNA expression by the electrolyzed hydrogen water (EHW) in human colorectal cancer cells (Caco-2 cells), with filtered water (ACW) as a reference. Similarly to FIG. 1, FIG. 5 indicates a statistical significance level on the vertical axis, and a higher value indicates higher statistical significance. The horizontal axis of FIG. 5 indicates the level of microRNA expression by the electrolyzed hydrogen water, and numerical values smaller than 0 represent microRNAs with decreased expression levels, while numerical values larger than 0 represent microRNAs with increased expression levels. FIG. 5 shows that 50 differentially expressed genes (DEGs) were extracted from the filtered water and the electrolyzed hydrogen water (extracted with p < 0.05 in FDR). Of the 50 genes, 27 genes are differentially expressed genes showing increased microRNA expression levels (hsa-miR-148a-3p, hsa-miR-215-5p, hsa-miR-192-5p, hsa-miR-27b-3p, hsa-miR-3529-3p, hsa-miR-206, etc.). On the other hand, 23 genes are differentially expressed genes showing decreased microRNA expression levels (hsa-miR-373-3p, hsa-miR-372-3p, hsa-miR-30d-5p, hsa-miR-200c-3p, hsa-miR-7-5p, hsa-miR-125a-5p, hsa-miR-7-5p, hsa-miR-516b-5p, etc.).

FIG. 6 is a heatmap showing, by color shading, the amount (degree) of microRNA expression changes caused by the electrolyzed hydrogen water (EHW) in human colorectal cancer cells (Caco-2 cells), with filtered water (ACW) as a reference. The heatmap compared two sets of independent experimental data by normalization. Alphabetical letters in FIG. 5 represent gene names. In FIG. 5, the microRNAs whose expression was increased by the electrolyzed hydrogen water (hsa-miR-192-5p to hsa-miR-200a-5p) are shown in the upper part of the table, and the microRNAs whose expression was decreased by the electrolyzed hydrogen water (hsa-miR-194-5p to hsa-miR-182-5p) are shown in the lower part.

### <Gene Prediction and Construction of Interaction Network>

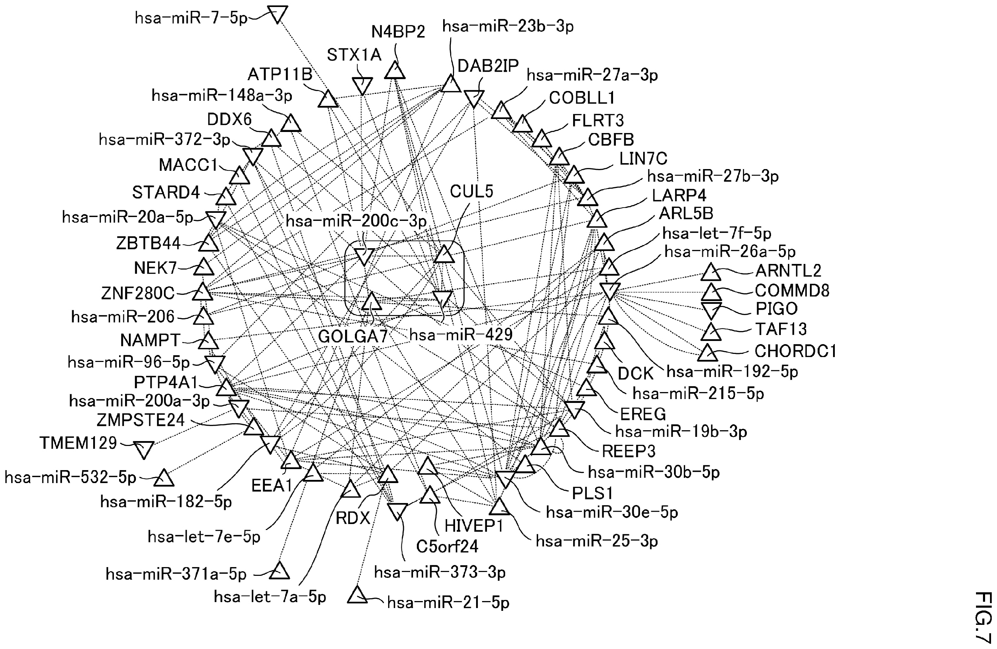

Based on TargetScan (https://www.targetscan.org/vert_72/), microRNA targets were predicted from the differentially expressed genes, and an mRNA-microRNA-related network was constructed. TargetScan is a microRNA target prediction algorithm and its database. MCODE was used to identify hub genes within the interaction network. MCODE is a technique for discovering protein complexes from a protein-protein interaction (PPI) network. FIG. 7 shows the results.

FIG. 7 shows the results of an analysis of mRNA-microRNA interaction by TargetScan and MCODE in human colorectal cancer cells (Caco-2 cells). Specifically, FIG. 4 shows the results of screening mRNA, which is a target of microRNA whose expression is changed by the electrolyzed hydrogen water (EHW), from the genes differentially expressed by the electrolyzed hydrogen water using TargetScan, and analyzing a protein-protein interaction (PPI) network using MCODE, that is, the network. From FIG. 7, CUL5 (Cullin 5), GOLGA7 (Golgin A7), has-miR-429, and has-miR-200c-3p were selected as hub genes. CUL5 and GOLGA7 are differentially expressed genes with increased expression. The has-miR-429 and has-miR-200c-3p are differentially expressed genes with decreased expression.

### <Prediction and Selection of Hub Gene that Regulates Transcription Factor (TF)>

TF was considered a gene in finding mRNA-microRNA interactions. The TF-mRNA and TF-microRNA pairs were found in the following three transcription factor databases:
hTFtarget (https://ngdc.cncb.ac.cn/databasecommons/database/id/6946);
motifmap (http://motifmap.ics.uci.edu/); and
TransmiR v2.0 (http://www.cuilab.cn/Transmir).

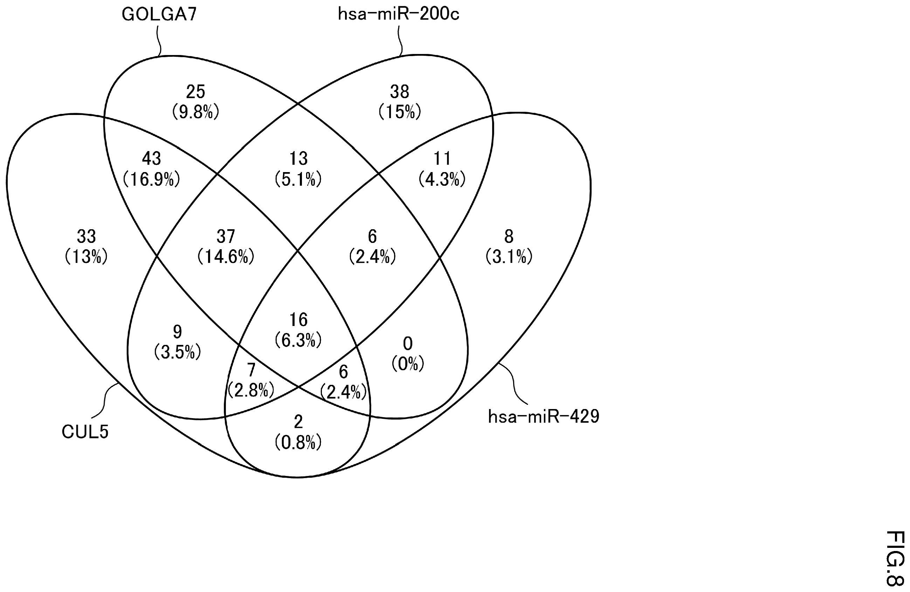

FIG. 8 shows the results.

FIG. 8 shows the results of the analysis of transcription factors common to hub genes responding to the electrolyzed hydrogen water (EHW) in human colorectal cancer cells (Caco-2 cells) using the three transcription factor databases. Specifically, FIG. 8 shows the results of the analysis of transcription factors common to the four hub genes identified above (CUL5, GOLGA7, has-miR-429, and has-miR-200c-3p). From FIG. 8, 16 transcription factors shown in Table 1 below were found as common transcription factors that regulate the four hub genes CUL5, GOLGA7, has-miR-429, and has-miR-200c-3p by comparison against the three transcription factor databases. Among these transcription factors, "KLF4" (Krueppel-Like Factor 4), in particular, is considered to be likely involved in the regulation of CUL5, GOLGA7, has-miR-429, and/or has-miR-200c-3p in the intestinal epithelium.

**[Table 1]**

| **symbol** | **gene name** |
|---|---|
| E2F1 | E2F transcription factor 1 |
| EP300 | E1A Binding Protein P300 |
| HDAC2 | Histone Deacetylase 2 |
| MAX | MYC Associated Factor X |
| MYB | MYB Proto-Oncogene |
| MYC | MYC Proto-Oncogene |
| SP1 | Sp1 Transcription Factor |
| TFAP4 | Transcription Factor AP-4 |
| EGR1 | Early Growth Response 1 |
| KDM5B | KDM5B lysine demethylase5B |
| FOXA1 | Forkhead box protein A1 (FOXA1) |
| AR | androgen receptor |
| KLF5 | KLF5 Kruppel like factor 5 |
| SRF | SRF serum response factor |
| **KLF4** | **Kruppel Like Factor 4** |
| TP53 | Tumor Protein P53 |

### <Summary>

Through the above analysis, the four responsive genes, CUL5 (Cullin 5), GOLGA7 (Golgin A7), has-miR-429, and has-miR-200c-3p, were identified as the hub genes responsive to the electrolyzed hydrogen water having a dissolved hydrogen concentration of 1,056 ppb or more and 1,080 ppb or less. The electrolyzed hydrogen water of the present invention acts on the enhancement of the intestinal barrier maintenance function through immune regulation, that is, has activity for enhancing the intestinal barrier maintenance function, by increasing or decreasing the expression of these responsive genes.

### INDUSTRIAL APPLICABILITY

The present invention is applicable as electrolyzed hydrogen water having activity for enhancing the intestinal barrier maintenance function.

## Claims

1. Electrolyzed hydrogen water having activity for enhancing an intestinal barrier maintenance function by regulating expression of a responsive gene, wherein
the responsive gene is at least one hub gene selected from the group consisting of CUL5 (Cullin 5), GOLGA7 (Golgin A7), has-miR-429, and has-miR-200c-3p.

2. The electrolyzed hydrogen water of claim 1, wherein
a concentration of dissolved hydrogen is 1,056 ppb or more and 1,080 ppb or less.

3. A method for enhancing and activating an intestinal barrier maintenance function by regulating expression of a responsive gene using electrolyzed hydrogen water, wherein
the responsive gene is at least one hub gene selected from the group consisting of CUL5 (Cullin 5), GOLGA7 (Golgin A7), has-miR-429, and has-miR-200c-3p, and
the method comprises adjusting a concentration of dissolved hydrogen to 1,056 ppb or more and 1,080 ppb or less.

4. A method for producing electrolyzed hydrogen water that enhances and activates an intestinal barrier maintenance function by regulating expression of a responsive gene, wherein
the responsive gene is at least one hub gene selected from the group consisting of CUL5 (Cullin 5), GOLGA7 (Golgin A7), has-miR-429, and has-miR-200c-3p, and
the method comprises adjusting a concentration of dissolved hydrogen to 1,056 ppb or more and 1,080 ppb or less.

5. A method for regulating gene expression using electrolyzed hydrogen water (excluding medical practice to humans), wherein
a gene responsive to the electrolyzed hydrogen water is at least one hub gene selected from the group consisting of CUL5 (Cullin 5), GOLGA7 (Golgin A7), has-miR-429, and has-miR-200c-3p, and
the method comprises: adjusting a concentration of dissolved hydrogen to 1,056 ppb or more and 1,080 ppb or less; and increasing expression of CUL5 (Cullin 5) and GOLGA7 (Golgin A7) and decreasing expression of has-miR-429 and has-miR-200c-3p by the action of the electrolyzed hydrogen water.
